Europäisches Patentamt

**European Patent Office**

**Office européen des brevets**

(19)

(11) Publication number: **0 068 318**
A1

# EUROPEAN PATENT APPLICATION

(12)

(21) Application number: **82105295.8**

(22) Date of filing: **16.06.82**

(51) Int. Cl.³: **A 61 F 5/44**

(30) Priority: **17.06.81 ES 503518**
**30.12.81 ES 509026**

(43) Date of publication of application: **05.01.83**
**Bulletin 83/1**

(84) Designated Contracting States: **AT BE CH DE FR GB IT
LI LU NL SE**

(71) Applicant: **Ingenieria Medica Aplicada, S.A. (IMASA),
Zaragoza, 2-4 Sitges, Barcelona (ES)**

(72) Inventor: **Renaga Sykes, Fernando, Aribau, 153,
Barcelona (ES)**

(74) Representative: **Lehn, Werner, Dipl.-Ing. et al, Hoffmann,
Eitle & Partner Patentanwälte
Arabellastrasse 4 (Sternhaus), D-8000 München 81 (DE)**

(54) **A prosthetic device for perineal enterostomy.**

(57) A prosthetic device for perineal enterostomy comprises
a first support (1), preferably a plate, for attachment to the
caudal area of the sacral bone.

A second support (2), attached to support (1), provides
a closure surface and an orifice for communicating with the
extremity (8) of an intestine (3) to be controllably closed.

A closure device (6, 7) provides a means of controllably
opening and closing the intestine (3) for evacuation thereof.

The closure device may comprise a magnetic ring (6)
cooperating with a magnetic plug (7).

## TITLE: A PROSTHETIC DEVICE FOR PERINEAL ENTEROSTOMY

The present invention relates to a prosthetic device for perineal enterostomy (colostomy or ileostomy).

As is already known, surgical operations for the removal of all or part of the large intestine require the provision of an artificial anus.

Traditionally the method used for the suppression of a part of the colon has taken the form of providing an aperture in the abdominal zone so as to allow for the evacuation from the intestine and for which aperture the necessary closure system or bags have to be provided.

However, traditionally, perineal colostomy (intestinal aperture in the perineum) from the outset of colonic surgery has been discarded, due to the total incontinency which arose therefrom and the impossibility of applying bags to collect the faeces, given the position and anatomical disposition of the part of the body under consideration.

This present invention has the object of overcoming the said difficulties, allowing the operation to be carried out in such a manner that an artificial anus can be provided in the same position as the natural anus and such that substantially total continency can be achieved.

According to the invention, there is provided a prosthetic device for perineal enterostomy, characterized by: a first support means for substantially rigid attachment to the caudal zone of the sacral bone; a further support means for determining the perineal plane and providing a closure surface and an orifice for communicating with the extremity of an intestine; and a closure device for mating with said closure surface.

The closure device of course allows controllable opening and closing of the extremity of the intestine concerned for evacuation thereof.

This prosthetic device preferably has as its fundamental basis the provision of a plate or blade, designed to coincide with the concavity of the sacral bone, and another support area, designed to receive the extremity of the intestine, which has to provide the outlet to the exterior,

-2-

such that the area of the plate or blade designed to be attached to the sacral bone has a form which is effectively coincident in so far as its shape is concerned with the caudal zone of the sacral bone in such a way that the area designed to support and hold the extremity of the intestine can have attached thereto a closure device of for example a magnetic or any other type.

The support means for attachment to the caudal zone of the sacral bone can be provided with orifices of various dimensions and arrangements so as to allow it to be attached to the sacral bone itself, this by means of pins or other rigid means, or it may be secured by the suture of its edges to the pre-sacral tissue thus providing the attachment of the prosthesis in a fixed position with respect to the body of the patient.

The variation in the concavity of the sacral bone from one individual to another can be provided for by the application of an acrylic cement between the sacral bone and the prosthesis.

The angle formed between the support for attachment of the prosthesis to the sacral bone and the support designed to carry the closure means is adaptable to the corresponding human anatomical angle and for this purpose there can be provided means to allow an adjustment of the relative position between the said first support means and the further support means, thus ensuring good adaptation to the body of the patient. This can be by means, for example, of hinges or other type of adjustable device.

The further support means will normally have an annular, elliptical or similar form which would provide for optimum passage for the faeces at the moment of opening and the whole assembly is preferably so dimensioned that once the first support means has been attached to the sacral bone, in the most suitable area, the zone for carrying the extremity of the intestine and the closure means will be positioned above the ischial tuberosities and in the same plane as was the original anus that had to be amputated. This will allow the patient carrying the prosthesis to assume a sitting position despite the closure device that he is having to have fitted.

The said support zone of the further support means will then have positioned within it a ring or magnetic closure of one of various shapes and arrangements which will permit the closing of the esterostomy by means of a plug or complementary device for its closure, this being by a magnetic or any other means.

For preference, the part of the caudal intestine be it the colon or ileum that has to open to the exterior will pass through the orifice in the further support as well as the magnetic ring if provided. The free mucous intestinal edge can then be secured to the surrounding skin by suturing, clips or other suitable means such that the prosthesis and its magnetic ring are completely hidden and do not communicate with the exterior.

This present invention covers a possible variation of the closure means of the perineal prosthesis consisting of two flattened closure areas joined by an intermediate stem which will be hollow, one of the said flattened areas being the internal closure means of the perineal prosthesis adapting itself to the lower part of the perineal aperture, this being achieved by prosthetic means, while the other

-4-

flattened component of closure provides the closure of the
perineal prosthesis on the outside thereof, thus causing a
very efficient double closure effect. This closure means
includes means for the evacuation of gases consisting of a
series of small channels which open lateraly on the edges of
the internal flattened closure area and communicate with a
central orifice or void provided in the stem which joins the
two flattened closure areas, opening to the exterior in the
lower area.

A characteristic of this closure means is that the
lower or exterior closure area is provided with adhesive
means for the purpose of its adaptation and sealing to the
skin, thus permitting it to offer a correct adjustment to
the anatomy of the patient of the assembly.

This closure means, made from a material of a
flexible consistency, allows for a simple positioning by the
introduction of a rod placed within the central orifice
which serves for the evacuation of gases, allowing a simple
withdrawal by pulling.

It is evident that the prosthesis should be made
from a biotolerable material, even of a classic type, such
as silicone rubber, polyethylene, epoxy resin, nylon,
polyurethane or other, metal such as titanium, or other
material.

Embodiments of the present invention will now be
described in more detail, by way of example, by reference to
the accompanying drawings in which:

Figures 1 and 2 show schematically respective views
of the human sacrum illustrating the attachment of one embodiment of prosthetic device, in accordance with this invention and the adaptation of the extremity of the large intestine to the same;

Figures 3 and 4 show respectively frontal views of one embodiment of the prosthesis in accordance with the present invention showing in the second case in schematic form the coupling of the intestine and the closure device;

Figure 5 shows a lateral elevation of the prosthesis in accordance with Figures 3 and 4;

Figure 6 shows the closure device, comprising possibly a magnetic ring and a closure plug;

Figure 7 shows schematically the disposition of a further embodiment of the prosthesis according to the invention after the operation carried out in order to fit it; and

Figures 8 and 9 are respectively views in perspective and in lateral elevation of the embodiment corresponding to Figure 7.

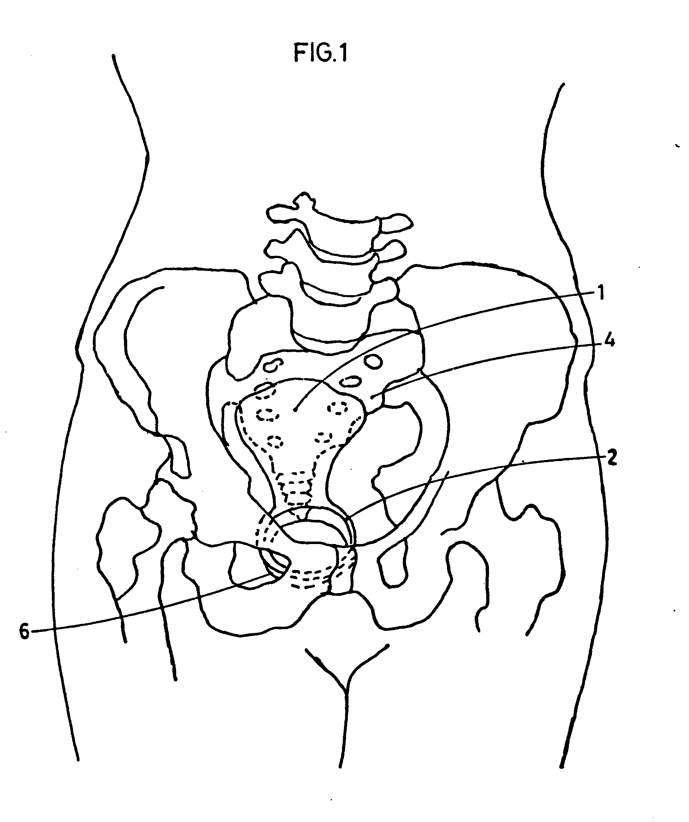

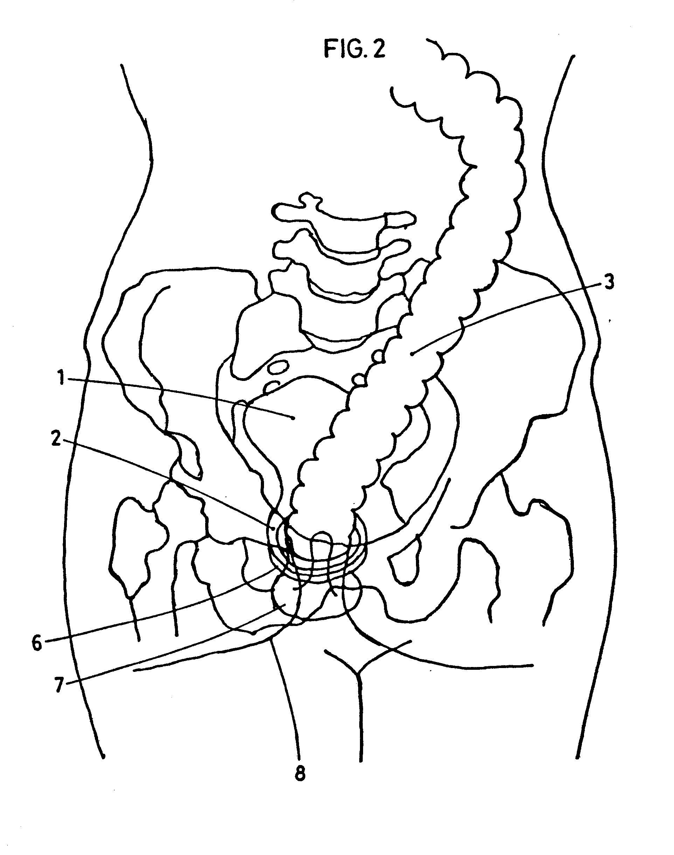

Referring to Figures 1 to 6, the illustrated prosthetic device, one embodiment of the present invention, comprises an upper support in the form of a plate or blade 1 having an attachment area designed to be fitted to the caudal part of the sacral bone, for which purpose the plate 1 takes an external shape effectively coincident with the profile of the sacral bone in the aforementioned caudal area. At the lower end of plate 1 is provided a support 2 designed to receive the extremity of the intestine 3, (see Figure 2) which latter can be suitably sutured or otherwise secured in position. The dimensions of the prosthesis are such that once the plate or expansion 1 has been attached to the sacral bone 4 in the caudal zone, the lower support 2 is located above the ischiatic tuberosities, leaving it positioned in a plane similar to that of the natural anus.

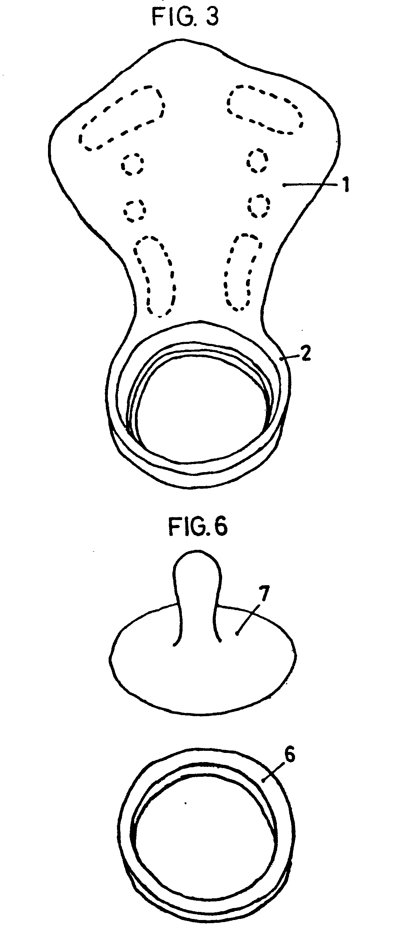

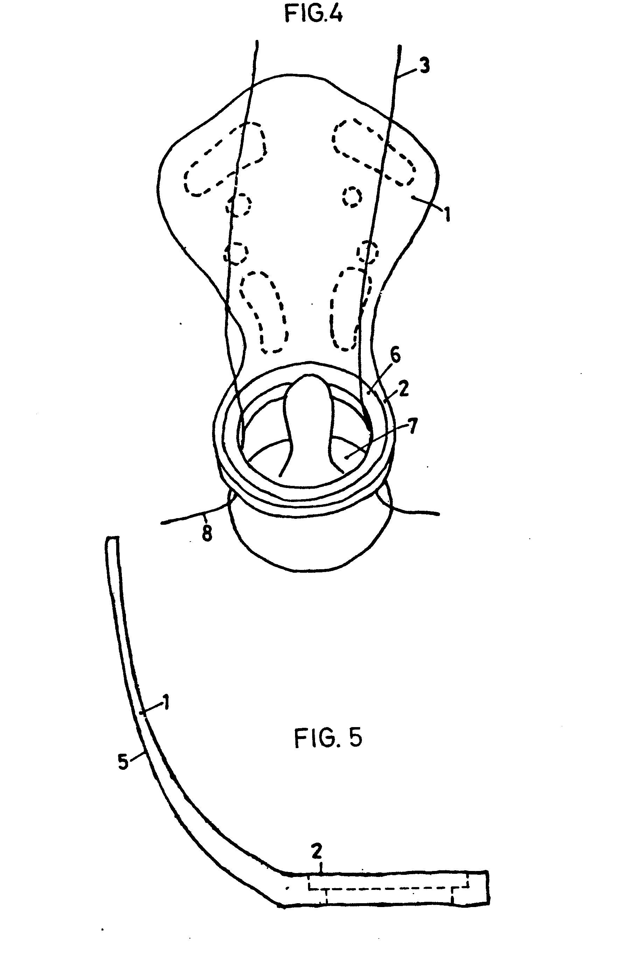

As may be seen in Figures 3 and 4 the expansion plate or blade 1 is of variable shape, given that characteristically its lateral elevation profile, as seen in Figure 5, offers at least on its external surface 5, a profile similar to that of the sacral bone to which it has to be joined. By this arrangement it is easy to adapt the prosthesis, as regards its external surface 5 referred to above, to the concave area of the caudal zone of the sacral bone and to secure it by means of pins, or other rigid system, passing through suitably arranged holes in the blade or expansion plate 1 or by means of sutures at the edges to the pre-sacral tissue.

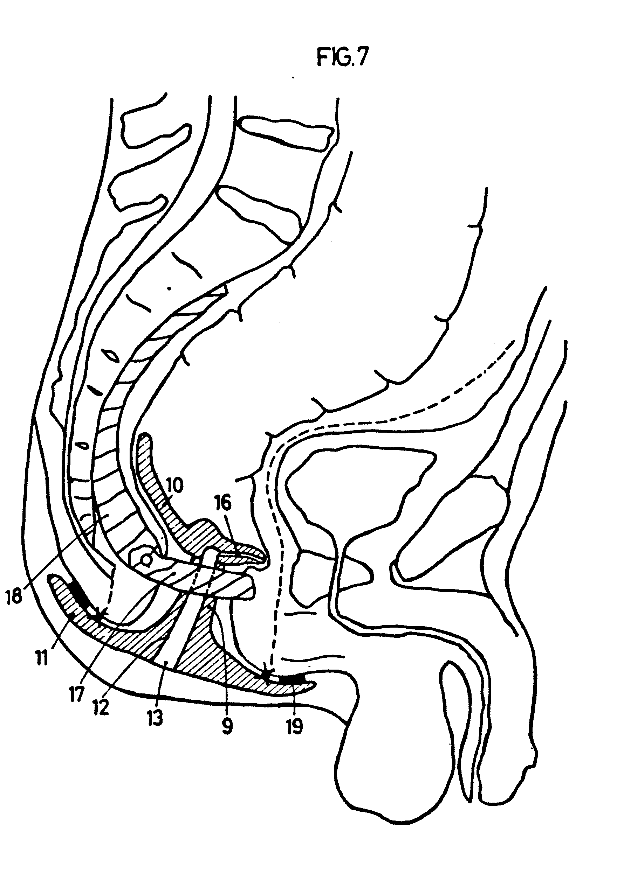

The angle formed between the attachment plate (upper support) 1 and the lower support 2 can be variable, so as to coincide with the angle formed effectively by the caudal extremity of the sacral bone and the perineal plane, in human anatomy. So as to permit of a better adjustment of the said angle to the patient's anatomy the prosthesis can be suitably adapted, in respect of its deformation characteristics, by means of hinges, as is shown in Figure 7, or by other means of connection between the plate 1 and the support 2 such that once the definitive position has been established one of the components can be attached to the other. The precise form of the support 2 will vary according to the means of closure to be utilized, in view of the need for the closure components to be adapted to each other.

Generally a circular shaped support would be provided, there being coupled to this (or any other shape of support) a magnetic-type ring 6 which will then accept a closure plug 7, equally with magnetic properties, thus providing the requisite closure of the assembly.

The precise form of coupling of the magnetic ring 6 as well as its shape can be widely varied.

In a preferred embodiment, the ring 6 is embedded in the interior of the material of support 2, the intestine wall passing through the orifice of support 2, permitting it to be sutured or otherwise secured to the surrounding skin at 8, thus achieving a highly efficient prosthesis in respect of the function that it has to fulfill.

In the embodiments shown in Figures 7 to 9 the device comprises a perineal closure prosthesis constituted primarily by an intermediate stem 9 which at the upper end is joined to a flattened body 10 for internal closure and at the lower end is joined to another flattened body 11, the upper expansion 10 taking the form of a flattened head with the appearance of a mushroom.

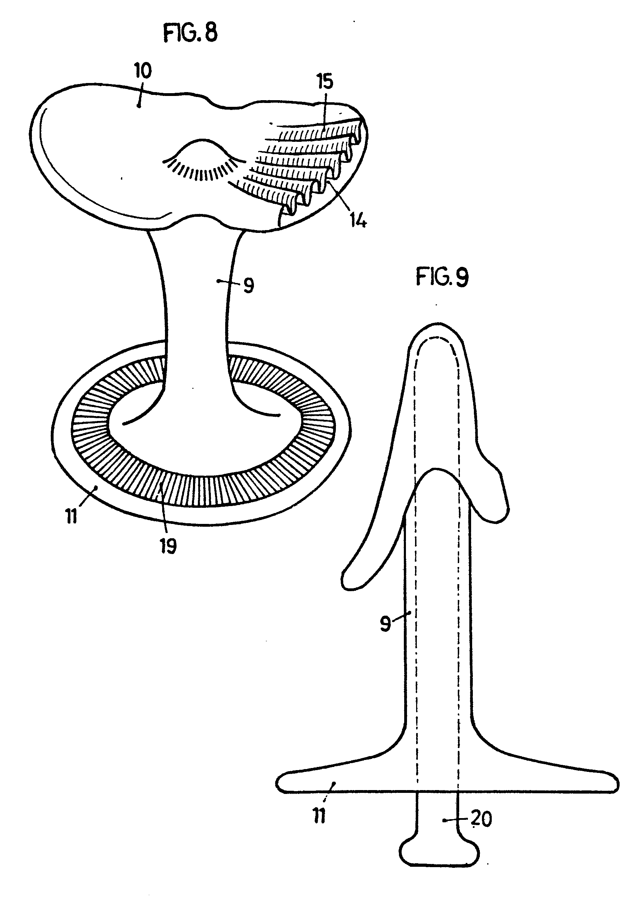

In accordance with this present invention the closure device described above may have within it an internal longitudinal channel 12 which at its lower end provides an aperture 13 and in which the upper end connects with a series of orifices 14 disposed laterally in the upper expansion 10 (Figure 8) these being for the purpose of allowing for the evacuation of gases.

-8-

While the orifices 14 can be provided in a variety of arrangements in the lateral zones of the expansion 10, in a preferred embodiment they would take the form shown in Figure 8 in which may be seen a multiplicity of folds 15 of the sheet of material from which the upper part of the expansion 10 has been formed, these determining the orifices 14 together with a series of channels 16 which communicate with the central orifice 12 (Figure 7).

In this version the perineal prosthesis 17 is preferably articulated by means of a hinge about the internal support 18.

The prosthetic closure is combined with the stump at the lower zone of the colon fixed to the perineum, the stump preferably passing through the prosthesis so as to form the perineal colostomy.

The prosthetic closure of Figures 7 to 9 comprises for preference an annular adhesive means 19 (Figure 8) which allows the prosthetic device to be affixed to the patient's skin as is shown in Figure 7.

The fitting of the prosthesis can be carried out easily by means of an ancilliary rod 20 (Figure 9) which fits into the interior of the channel 12 thus providing an easy insertion of the prosthesis into the position of utilization or closure.

This colostomy closure is placed in its position of service by means of the ancilliary rod 20 which pushes against the head 10 of the prosthesis, which latter is deformed to pass through the attachment ring of the colostomy. Having passed the said ring obstacle, the head 10 re-assumes its normal shape, preventing the escape of the faeces by blocking the ring, allowing solely the exit of gases through the orifices 14, the internal channel 12 and the aperture 13. The weight of the faeces themselves contributes to the closing of the said head 10.

Extraction of the prosthetic system is carried out by pulling it in the opposite direction to that of insertion, causing the head 10 to be forceably deformed in the opposite sense to that of its introduction so as to enable it to pass through the fixing ring of the colostomy.

As may be understood, major modifications can be incorporated in the illustrated prosthetic devices without departing from the scope of the invention, as for example providing a closure device in the form of an expandable body designed to remain in the inside of the prosthesis and which can be controlled by means of a small tube and an external valve.

Also the arrangement of the orifices for the evacuation of the gases may be a variable as long as they communicate with the open ended central passage.

The closure means in this variant can be combined with the fitting of an active carbon element in the interior of the longitudinal inner channel 12 within the stem 9 so as to absorb the bad odours of the gases.

Furthermore, the prosthetic closure means described above can be manufactured from materials which would be suitable for its production and sale at a low cost with a view to its utilization on a disposable basis.

All changes which do not affect, alter, change or modify the essentiality of the prosthetic device herein described, is within the scope of protection afforded by the appended claims.

CLAIMS

1. A prosthetic device for perineal enterostomy, characterized by: a first support means (1, 18) for substantially rigid attachment to the caudal zone of the sacral bone (4); a further support means (2, 17) for determining the perineal plane and providing a closure surface and an orifice for communicating with the extremity (8) of an intestine; and a closure device (7, 10) for mating with said closure surface.

2. A prosthetic device for perineal enterostomy as claimed in claim 1, characterized in that the angle between the first support means and the further support means corresponds to the angle between the caudal zone of the sacral bone and the human perineal plane.

3. A prosthetic device for perineal enterostomy as claimed in claim 1 or 2 wherein the relative angle between the first support means (1; 18) and the further support means (2, 17) is adjustable within specific limits.

4. A prosthetic device for perineal enterostomy as claimed in claim 3 wherein the further support means (2, 17) is articulated to the first support means (1, 18), the articulation being capable of being fixed once the angle has been set.

5. A prosthetic device for perineal enterostomy, as claimed in any one of claims 1 to 4 wherein the height of the first support means (1, 18) is such that in use the further support means will be located above the ischial tuberosities.

6. A prosthetic device for perineal enterostomy, as claimed in any one of claims 1 to 5 wherein the first support means (1, 18) has a surface formed in such manner that it mates with the surface of the sacral cavity to which it has to be attached.

7. A prosthetic device for perineal enterostomy, as claimed in any one of claims 1 to 6 characterized by the provision of orifices and holes in the first support means for allowing it to be fixed by means of pins or similar rigid elements or by sutures to the sacral bone or to the pre-sacral tissue.

8. A prosthetic device for perineal enterostomy, as claimed in any one of the preceding claims, characterized in that said closure device is a double closure device, formed from flexible material, and provided with a flattened head (10) designed to coincide with, and effect the closure of the perineal prosthesis from the interior of the relevant intestine and being joined to a lower expansion (11) by means of a connecting stem (9), the said lower expansion (11) being also flattened and providing an external closure, in use, in the area adjacent to the colostomy.

9. A prosthetic device for perineal enterostomy, as claimed in claim 8, in which the connecting stem (9) joining the two closure areas (10, 11) at its extremities

-12-

is provided with an internal channel (13) which at its upper end communicates with a series of orifices (14) which open laterally in said flattened head (10) so as to provide for the evacuation of gases, and at its lower end provides a free outlet.

10. A prosthetic device for perineal enterostomy, as claimed in claim 9, in which said series of orifices (14) is formed by an arrangement of folds (15) determining a multiplicity of channels in the upper part of the internal closure head, these channels communicating with the internal channel (13) in the stem (9).

11. A prosthetic device for perineal enterostomy as claimed in claim 8, 9, or 10 characterized by the provision of an annular zone (19) of adhesion in the upper face of the external closure expansion so as to enable it to be attached to the skin of the user.

- 1/4 -

FIG.1

0068318

- 2/4 -

FIG. 2

FIG. 3

FIG. 6

0068318

FIG.4

FIG. 5

- 5/16 -

FIG.7

- 6/6 -

FIG. 8

FIG. 9

0068318

European Patent
Office

**EUROPEAN SEARCH REPORT**

Application number

EP 82105295.8

| | DOCUMENTS CONSIDERED TO BE RELEVANT | | |
|---|---|---|---|
| Category | Citation of document with Indication, where appropriate, of relevant passages | Relevant to claim | |
| A,P | GB – A – 2 070 936 (STEER) | | |
| | -- | | |
| A | US – A – 4 067 335 (SILVANOV) | | |
| | -- | | |
| A | US – A – 3 548 828 (VASILE) | | |
| | -- | | |
| A | FR – A1 – 2 326 208 (CLAYTON) | | |
| | -- | | |
| A | US – A – 3 802 418 (CLAYTON) | | |
| | ---- | | |

**CLASSIFICATION OF THE APPLICATION (Int. Cl. 3)**

A 61 F 5/44

**TECHNICAL FIELDS SEARCHED (Int.Cl. 3)**

A 61 F 5/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| X | The present search report has been drawn up for all claims | |
|---|---|---|
| Place of search VIENNA | Date of completion of the search 14–10–1982 | Examiner EBERLE |

EPO Form 1503.1  06.78